# EUROPEAN PATENT APPLICATION

(11) **EP 4 317 991 A1**
(43) Date of publication of application: **07.02.2024**
(21) Application number: 22781219.5
(22) Date of filing: 31.03.2022
(51) Int. Cl.: G01N 37/00, G01N 33/53, G01N 33/543

(54) **HIGHLY SENSITIVE IMMUNOASSAY REAGENT AND MEASUREMENT METHOD**

(30) Priority: 31.03.2021 JP 2021060870
(71) Applicant: LSI Medience Corporation, Tokyo 105-0023 (JP)
(72) Inventor: MATSUSHITA, Shuhei, Tokyo 105-0023 (JP)
(74) Representative: Gille Hrabal Partnerschaftsgesellschaft mbB Patentanwälte
(86) International application number: PCT/JP2022/016460
(87) International publication number: WO 2022/211009

(57) **Abstract**

Provided are a highly sensitive measurement reagent and a measurement method to make it possible to accurately and conveniently measure the sCLEC-2 concentration in blood even at the level of healthy persons. The measurement reagent contains a carrier on which a first immunological partner that binds to a physiologically active substance contained in a biological sample is immobilized; a second immunological partner that binds to the physiologically active substance; and an amphiphilic substance that contains, as a hydrophobic portion, a linear, branched, or cyclic saturated or unsaturated hydrocarbon group with 6 to 12 carbons, an aromatic hydrocarbon group with 6 to 12 carbons, or a steroid skeleton; or a chaotropic substance.

## Description

### TECHNICAL FIELD

The present invention relates to a reagent and a method for highly sensitive measurement of a substance in biological samples.

### BACKGROUND ART

Currently, in the field of clinical diagnostic testing, it is required to measure a wide variety of substances that can be used as indicators of disease diagnosis in a short time with high accuracy, and to feed back the results quickly and accurately to the field of treatment. For example, accurate quantification of trace amounts of substances to be measured is often performed by measurement systems in which physiologically active substances (proteins such as antibodies, enzymes, receptors, and the like, antigens, nucleic acids such as DNA, RNA, and the like, sugar chains, or the like) are attached to the particle surface, or by immunological measurements using antigen-antibody reactions.

In general, the performance of in vitro diagnostics used for clinical diagnostic testing is highly dependent on the performance of the main raw materials. There are two main raw materials. One is a physiologically active substance, represented by an antibody that recognizes the target substance, and the other is a particle that carries the physiologically active substance. However, it is not possible to construct a sufficient measurement method only with the physiologically active substance and particles that have the desired reactivity and properties.

In in vitro diagnostic reagents utilizing antigen-antibody reaction using the antibody, in addition to physiologically active substances such as antibodies and particles carrying such physiologically active substances, various additives such as surfactants, salts, human antibodies, preservatives, and sensitizers are used, and these additives are also factors that affect the performance of the diagnostic reagents.

As an example of a reagent that requires such a highly accurate measurement, an sCLEC-2 measurement reagent is described below. C-type lectin-like receptor 2 (CLEC-2) has been identified on platelets as a receptor for platelet-activating snake venom rhodocytin. CLEC-2 is expressed almost exclusively on platelets and megakaryocytes in humans, making it a platelet-specific molecule. It was reported that CLEC-2 is released into the blood as soluble CLEC-2 (soluble CLEC-2, hereinafter referred to as sCLEC-2) when platelets are activated. (Patent literature 1, Non-patent literature 1).

When the blood sCLEC-2 concentration is high, it may indicate some pathological condition, and a highly sensitive and specific measurement method is indispensable to quickly identify such a situation.

Although sCLEC-2 in blood has been measured by ELISA (Patent literature 1) (Non-Patent literatures 1 to 3), the sensitivity of the ELISA method is not sufficient, and there are still issues to be solved in terms of practical use in clinical site. For example, in Patent literature 1, the sample is diluted 5-fold in consideration of the influence of inhibitors existing in blood.

### CITATION LIST

### PATENT LITERATURE

[Patent literature 1] JP 2014-070942 A
[Patent literature 2] Japanese Patent Application No. 2021-003671

### NON-PATENT LITERATURE

[Non-patent literature 1] F. Kazama et al., Platelets 2015; 26 (8) : 711-719
[Non-patent literature 2] Y. Yamashita et al., Thrombosis Research 178 (2019) 54-58
[Non-patent literature 3] X. Zhang et al., Stroke.2019;50:45-52

### SUMMARY OF INVENTION

### TECHNICAL PROBLEM

Measurement of sCLEC-2 in blood has been shown to be useful in the diagnosis of cerebral infarction, myocardial infarction, deep vein thrombosis, pulmonary thromboembolism, thrombotic microangiopathy, disseminated intravascular coagulation syndrome, and the like. These diseases are very serious, often requiring intensive care, and have a high mortality rate, so early diagnosis is important. In order to accurately diagnose these diseases, it is most important to be able to clearly distinguish between healthy persons and thrombotic patients, and for this reason it is extremely important to be able to more accurately measure sCLEC-2 concentrations in the blood of healthy persons.

The measurement of blood sCLEC-2 concentration has been suggested to be useful not only for diagnosis of diseases but also for monitoring therapeutic effects and judgement of prognosis (Patent literatures 1 and 2). Administration of antiplatelet agents to patients with thrombosis is expected to reduce the blood sCLEC-2 concentration to the range of sCLEC-2 concentration in healthy persons. Therefore, it is very important to be able to correctly measure sCLEC-2 in the concentration range of healthy persons, even for the purpose of monitoring the therapeutic effect in patients with thrombosis.

The above-mentioned applications for diagnosis, monitoring of therapeutic effects, and judgement of prognosis are possible by more accurate measurement of sCLEC-2 concentrations in blood of healthy persons. Therefore, an object of the present invention is to provide a highly sensitive measurement reagent and a measurement method that enable accurate and easy measurement of sCLEC-2 concentration in blood even at the level of healthy persons.

### SOLUTION TO PROBLEM

In order to accurately measure sCLEC-2 concentrations in blood of normal persons, it is necessary first to increase the sensitivity of the measurement so that lower concentrations of sCLEC-2 can be measured. In addition, it is necessary to eliminate the influence of interfering substances on sCLEC-2 present in the blood so that accurate sCLEC-2 concentrations can be measured.

The present inventor has made intensive studies to solve the aforementioned problem. As a result, the present inventor found a method to measure blood sCLEC-2 with high sensitivity by adding an amphiphilic substance having a linear, branched, or cyclic saturated or unsaturated hydrocarbon group with 6 to 12 carbons, an aromatic hydrocarbon group with 6 to 12 carbons, or a steroid skeleton in the hydrophobic portion to the measurement system for sCLEC-2 in blood, and completed this invention. In particular, it was surprising that amphiphilic substances having a relatively short linear, branched, or cyclic saturated or unsaturated hydrocarbon group with 6 to 12 carbons, an aromatic hydrocarbon group with 6 to 12 carbons, or a steroidal skeleton showed the above effects, while those with a carbon number of 13 or more (excluding the steroidal skeleton) did not show the above effects.

It was also found that the addition of these amphiphilic substances at relatively high concentrations of 0.05% to 5.33%, preferably 0.27% to 3.73%, and more preferably 1.07% to 2.67%, showed remarkable effects.

The present invention provides the following:
[1] A reagent for measuring a physiologically active substance, comprising:
   a carrier on which a first immunological partner that binds to the physiologically active substance contained in a biological sample is immobilized;
   a second immunological partner that binds to the physiologically active substance; and
   an amphiphilic substance that contains, as a hydrophobic portion, a linear, branched, or cyclic saturated or unsaturated hydrocarbon group with 6 to 12 carbons, an aromatic hydrocarbon group with 6 to 12 carbons, or a steroid skeleton; or a chaotropic substance.
[2] The reagent of [1], wherein the amphiphilic substance that contains, as a hydrophobic portion, a linear, branched, or cyclic saturated or unsaturated hydrocarbon group with 6 to 12 carbons, an aromatic hydrocarbon group with 6 to 12 carbons, or a steroid skeleton is a compound that contains any one of an anionic functional group, a cationic functional group, an amphiphilic functional group, a pyranoside group, a glucamine group, an oxyethylene group, or an aromatic ring which may be substituted.
[3] The reagent of [1] or [2], wherein two or more of the amphiphilic substances, or a combination of the amphiphilic substance with the chaotropic compound, is used.
[4] The reagent of any one of [1] to [3], wherein a concentration of the amphiphilic substance in a reaction liquid is 0.05% to 5.33%, and a concentration of the chaotropic compound in the reaction liquid is 0.27 mol/L to 3.2 mol/L.
[5] The reagent of any one of [1] to [4], wherein an item to be measured is soluble CLEC-2.
[6] A method of measuring a physiologically active substance contained in a biological sample using the reagent of any one of [1] to [5], comprising:
   (1) reacting the physiologically active substance contained in the biological sample with the carrier on which the first immunological partner that binds to the physiologically active substance contained in the biological sample is immobilized, and the second immunological partner that binds to the physiologically active substance, in the presence of the amphiphilic substance;
   (2) separating the carriers from substances other than the carriers in a reaction liquid after step (1); and
   (3) measuring the second immunological partner on the carrier that is separated in step (2).

### ADVANTAGEOUS EFFECTS OF INVENTION

The measurement reagent and the measurement method of the present invention enable highly sensitive measurement without the influence of interfering substances in the blood, thus making it possible to accurately measure the concentration of the substance to be measured in the blood of healthy persons. Therefore, accurate diagnosis, monitoring of therapeutic effects, and prognosis prediction become possible.

### BRIEF DESCRIPTION OF THE DRAWINGS

[Fig. 1] Figure 1 is a standard curve prepared using a human sCLEC-2 protein as a standard.
[Fig. 2] Figure 2 shows the dilution linearity obtained using a CLEIA method.

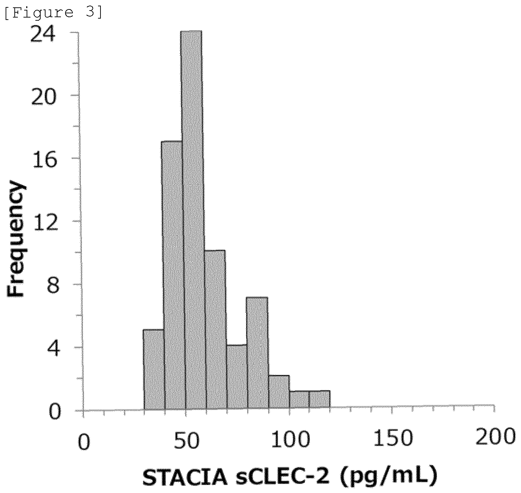

[Fig. 3] Figure 3 shows the distribution of sCLEC-2 in healthy persons obtained using a CLEIA method.

### DESCRIPTION OF EMBODIMENTS

The substance to be measured in the present invention is not particularly limited, as long as it can be measured by immunoassay. For example, soluble CLEC-2, soluble interleukin-2 receptor, prostate-specific antigen, cardiac troponin, presepsin, brain natriuretic peptide, human brain natriuretic peptide precursor N-terminal fragment, myoglobin, CK-MB, D-dimer, thrombin-antithrombin complex, estrogen, progesterone, human chorionic gonadotropin, luteinizing hormone, follicle stimulating hormone, prolactin, testosterone, procalcitonin, CRP, thrombomodulin, thyroid stimulating hormone, FT4, FT3, and the like may be exemplified, but the substance to be measured is not particularly limited, as long as it can be measured by immunological heterogeneous assay.

Hereinafter, the embodiments of the present invention will be described in detail using an sCLEC-2 measurement as an example, but the embodiments of use are not limited thereto.

The term "CLEC-2" as used herein is a platelet-activating receptor belonging to a C-type lectin family, which is usually present on the membrane of platelets and is released into the blood upon platelet activation. The term "soluble CLEC-2 (sCLEC-2)" as used herein means sCLEC-2 or sCLEC-2-derived molecules that are released from such platelets and detected in blood (or buffer if incubated in buffer).

sCLEC-2 includes proteins with molecular weights of approximately 40 kDa, approximately 32 kDa, approximately 25 kDa, and the like in SDS-polyacrylamide gel electrophoresis (SDS-PAGE) under reducing conditions (Patent literature 1). The proteins with molecular weights of approximately 40 kDa and approximately 32 kDa are present on the membrane surface of platelets, and it is presumed to be released in the form of being included in microparticles produced upon platelet activation. These are thought to have sugar chains attached to them. On the other hand, the protein with a molecular weight of approximately 25 kDa are thought to be cleaved by proteases and released from platelets upon platelet activation. In the present invention, the amount of sCLEC-2 as described above is measured. sCLEC-2 can be detected together as the proteins with molecular weights of approximately 40 kDa, approximately 32 kDa, and approximately 25 kDa, or it can be detected only as the protein with a molecular weight of approximately 25 kDa.

The CLEC-2 that can be used in the present invention has been identified in a wide range of animal species, including humans, cattle, pigs, guinea pigs, rats, mice, and the like, but human CLEC-2 is particularly preferred. The amino acid sequence may contain one to several substitutions due to race or the like, and the CLEC-2 to be detected may contain one to several substitutions (for example, five, preferably two).

Blood, plasma containing anticoagulants, and whole blood containing anticoagulants are preferred as samples (specimens) used in the present invention, but any biological fluids such as urine, spinal fluid, ascites fluid, pleural fluid, amniotic fluid, nasal fluid, tear fluid, or the like, and culture cell medium, throat and nasal swab, platelet-suspended plasma, or the like are also eligible for measurement.

The reagent of the present invention is not particularly limited, as long as it is used in a method of detecting a substance to be measured, but immunological methods using antibodies that recognize the substance to be measured are preferred. As the methods in which a protein is immunologically detected, any of the following methods which are commonly used can be used: for example, immunoassays using labeled antibodies, such as enzyme-linked immunoassay (ELISA), radioimmunoassay, immunochromatography, and the like, or Western blotting under non-denaturing conditions, latex agglutination, immunoturbidimetric method, chemiluminescence and enzyme immunoassay (CLEIA), chemiluminescence immunoassay (CLIA), electrochemiluminescence immunoassay (ECLIA), and the like. Among these methods, chemiluminescence and enzyme immunoassay (CLEIA), electrochemiluminescence immunoassay (ECLIA), chemiluminescence immunoassay (CLIA), and fluorescence immunoassay using labeled antibodies are preferably used in terms of high sensitivity, and those skilled in the art can select and use an appropriate method, taking into consideration the measurement equipment needed to achieve the measurement.

The immunological partner that can be used in the present invention may be a partner that immunologically and specifically binds to a substance to be measured, for example, an immunological substance (i.e., an antigen or an antibody) that can specifically binds to the above-mentioned various proteins, polysaccharides, lipids, nucleic acids, haptens, complexes or fragments thereof, or the like. When the immunological partner is an antibody, the antibody used may be a monoclonal antibody or a polyclonal antibody, or a fragment obtained by treating these antibodies with an enzyme or the like. The fragment of an antibody is preferably a functional fragment containing an antigen binding region or its variable region of the antibody, such as F(ab')₂, Fab', Fab, or the like. F(ab')₂ and Fab' are products obtained by treating immunoglobulin with a proteolytic enzyme (for example, pepsin, papain, or the like), and are antibody fragments produced by digestion before or after the disulfide bond between the two H chains in the hinge region. Immunological partners may be used in combination of multiple types.

The antibody that reacts with a substance to be measured is characterized by recognizing an antigenic determinant contained in the substance to be measured, and reacting with it. When the substance to be measured is sCLEC-2, such an antibody can be one that commonly recognizes the proteins with molecular weights of approximately 40 kDa, approximately 32 kDa, and approximately 25 kDa, or an antibody that recognizes only the protein with a molecular weight of approximately 25 kDa can be selected from antibodies produced by hybridomas and used.

The anti-CLEC-2 antibody can be produced, for example, by using a polypeptide containing part or all of the amino acid sequence of sCLEC-2 as an immunogen. The antigen polypeptide may be a synthetic polypeptide chemically synthesized according to a known method, or a polypeptide produced by genetic recombination or the like. Samples used for measurement are preferably of human origin, but samples derived from animals other than humans may be used to understand the pathology of experimental animals. Experimental animals are not particular limited, but for example, guinea pigs, rats, mice, dogs, and the like may be exemplified.

The antibody used in the present invention may be used as an immobilized antibody carried on an insoluble carrier such as a solid-phase carrier or the like, or as a labeled antibody labeled with a labeling substance.

The immobilized antibody is an antibody that are carried on an insoluble carrier by physical adsorption, chemical bonding, or the like. The immobilized antibody may be used to detect or quantify the substance to be measured contained in samples. Such insoluble carriers that can be used to carry antibodies include, for example, polymer materials such as latex, rubber, polyethylene, polypropylene, polystyrene, styrene-butadiene copolymer, polyvinyl chloride, polyvinyl acetate, polyacrylamide, polymethacrylate, styrene-methacrylate copolymer, polyglycidyl methacrylate, acrolein-ethylene glycol dimethacrylate copolymer, polyvinylidene difluoride (PVDF), silicone, and the like; agarose; gelatin; red blood cells; inorganic materials such as silica gel, glass, inert alumina, magnetic materials, etc. Inorganic materials such as silica gel, glass, inert alumina, magnetic materials, and the like; and the like. These may be used alone or in combination of two or more types.

The labeling substance is not particularly limited as long as it is a labeling substance that can be used in conventional immunological assays. For example, enzymes, fluorescent substances, radioisotopes, insoluble granular materials, and the like may be exemplified. As the enzymes for labeling, alkaline phosphatase, peroxidase, glucose oxidase, tyrosinase, acid phosphatase, and the like may be exemplified. As the fluorescent substances, fluorescein isothiocyanate (FITC), green fluorescent protein (GFP), luciferin, and the like may be exemplified. As the radioisotopes, ¹²⁵I, ¹⁴C, ³²P, and the like may be exemplified.

When the labeling substance is an enzyme, the labeling substance can be measured by performing a luminescence, fluorescence, or color reaction using a substrate for the enzyme. For example, when the enzyme is alkaline phosphatase, as the substrate, chemiluminescent substrates such as CDP-star (registered trademark)(disodium 2-chloro-5-(4-methoxyspiro{1,2-dioxetane-3,2'-(5'-chloro)-tricyclo[3.3.1.1^{3,7}Jdecane}-4-yl)-1-phenyl phosphate), CSPD (registered trademark)(disodium 3-(4-methoxyspiro{1,2-dioxetane-3,2-(5'-chloro)tricyclo[3.3.1.1.^{3,7}]decane}-4-yl)phenyl phosphate), AMPPD (registered trademark)(adamantyl methoxyphenylphosphoryl dioxetane, APS-5, and the like; fluorescent substrates such as 4-methylumbelliferylphosphate and the like; and chromogenic substrates such as p-nitrophenyl phosphate, BCIP (5-bromo-4-chloro-3-indolyl-phosphate), NBT (4-nitroblue tetrazolium chloride), INT (iodonitrotetrazolium), and the like may be exemplified.

The immunological measurement method of the present invention can be performed using an immunological measurement reagent consisting of one liquid or two or more liquids.

When the reagent of the present invention consists of one liquid, for example, it may consist of a reaction reagent containing at least an insoluble carrier carrying an immunological partner to form an immunological complex. According to known methods, the sample is reacted with the reagent and the signal is detected. The amphiphilic substance used in the present invention may be added to the reagent before the sample is reacted with the reagent, but can preferably be supplied as an additive to the reagent.

When the reagent of the present invention consists of two or more liquids, for example, it may consist of a stabilizing reagent, and a reaction reagent containing at least an insoluble carrier carrying an antibody or an antigen to form an immunological complex. The stabilizing reagent is a reagent that dilutes samples to an appropriate concentration or performs pretreatment, and can be prepared according to known methods. According to known methods, the sample is reacted with the stabilizing reagent, and then, reacted with the reaction reagent, and the signal is detected. The amphiphilic substance used in the present invention may be added to the stabilizing reagent and/or the reaction reagent before the sample is reacted with the reaction reagent, but can preferably be supplied as an additive to the stabilizing reagent and/or the reaction reagent.

The amphiphilic substance used in the present invention may be contained in the stabilizing reagent or in the reagent containing the insoluble carrier, as long as it may be in a form that will eventually be miscible with the sample.

The present invention found that the addition of the amphiphilic substance to the reagent within a certain concentration range is essential to achieve high sensitivity of the measurement system. This phenomenon is thought to be due to the physicochemical properties of the substance to be measured. However, when the amount of the amphiphilic substance is small, sufficient signal cannot be obtained for the highly sensitive measurement system.

The amphiphilic substance is only required to be contained in the reaction liquid in which the immunoreaction with the substance to be measured takes place. For example, it may be added to any or each of the following reagents: a diluent liquid to dilute measurement samples in advance, a reagent in which the immobilized antibody that binds to the insoluble carrier is mixed with the measurement sample to react the substance to be measured with the immobilized antibody, and a reagent in which the labeled antibody is mixed and reacted with the measurement sample to react the labeled antibody with the substance to be measured.

The amphiphilic substance is generally a molecule that has both hydrophobic and hydrophilic portions. In the present invention, the hydrophobic portion can be a relatively short carbon chain from C6 to C12. Generally, amphiphilic substances with long carbon chains, which are used in reagents for measuring substances to be measured contained in biological samples using immunoreaction, are not suitable. The hydrophobic portion can be a linear, branched, or cyclic saturated or unsaturated hydrocarbon group with a carbon chain of C6 to C12, an aromatic hydrocarbon group with a carbon chain of C6 to C12, or a steroid skeleton.

The hydrophilic portion of the amphiphilic substance may be anionic, cationic, nonionic, or amphiphilic, and may be a compound having any of the following: an anionic functional group, a pyranoside group, a glucamine group, an oxyethylene group, and an aromatic ring that may be substituted (in particular, an aromatic ring that is substituted with one or more hydroxyl groups).

Those with the anionic functional group include, but are not limited to, sodium octanoate, sodium n-nonanoate, sodium 1-pentanesulfonate, sodium 1-hexanesulfonate, sodium 1-heptanesulfonate, and sodium 1-octanesulfonate.

Those with the cationic functional group include, but are not limited to, n-octylamine hydrochloride, n-octyltrimethylammonium chloride, and decyltrimethylammonium chloride.

Amphiphilic substances of amphiphilic nature may also be used. The amphiphilic substances of amphiphilic nature include, but are not limited to, dimethyl(n-octyl)(3-sulfopropyl)ammonium hydroxide intramolecular salt, decyldimethyl(3-sulfopropyl)ammonium hydroxide intramolecular salt, CHAPS (3-[(3-cholamidopropyl)dimethylammonio] propanesulfonate).

Furthermore, amphiphilic substances with a branched hydrophobic structure may also be used. For example, amphiphilic substances with a branched structure include, but are not limited to, sodium 2-ethylhexyl sulfate.

Those with the pyranoside group can be any glycoside of pyranose, and the pyranoses include ribopyranose, arabinopyranose, xylopyranose, lixopyranose, allopyranose, altropyranose, glucopyranose, mannopyranose, gulopyranose, idopyranose, galactopyranose, and thalopyranose. Specifically, those with the pyranoside group include, but are not limited to, for example, N-octyl-β-D-glucopyranoside, n-heptyl-β-D-thioglucopyranoside, N-octyl-β-D-maltopyranoside, n-nonyl-β-D-thiomaltopyranoside, N-decyl-β-D-maltopyranoside, n-octanoyl-N-methyl-D-glucamine, α-D-glucopyranosyl-α-D-glucopyranoside monooctanoate, α-D-glucopyranosyl-α-D-glucopyranoside monodecanoate, and α-D-glucopyranosyl-α-D-glucopyranoside monodododecanoate

Those with the glucamine group may be those with the structure of N-methyl-D(-)-glucamine, and include, but are not limited to, n-nonanoyl-N-methyl-D-glucamine and n-octanoyl-N-methyl-D-glucamine.

Those with the oxyethylene group are those with an oxyethylene group having ethylene glycol as the basic structure include, but are not limited to, tetraethylene glycol monooctyl ether and poly(ethylene glycol) octyl ether.

Those with a cyclic skeleton include, but are not limited to, aromatic rings that may be substituted, for example, those with the structure of sodium salicylate as aromatic rings, and cholic acid and CHAPS (3-[(3-cholamidopropyl)dimethylammonio]propanesulfonate) as those with the steroidal group.

The term "amphiphilic substance" that can be used in the present invention is used as a generic term for compounds with both hydrophilic and hydrophobic portions, including surfactants (hereinafter referred to as the surfactant). The surfactant is not limited to those described above, and those skilled in the art can select and use substances with appropriate reactivity.

The amphiphilic substance aggregates in water to form bilayers represented by micelles and lipid bilayers. It functions as a surfactant by adsorbing at the interface between the aqueous and organic phases, lowering the surface tension, forming micelles, and uniformly mixing to form emulsions. This property may be used in a reagent to prevent antigen adsorption on insoluble carriers or to achieve nonspecific inhibition by providing a stronger washing effect.

In general, the properties of amphiphilic substances are determined by the balance between hydrophilic and hydrophobic portions, chain length, and bulk height. Amphiphilic substances are classified according to the nature of their hydrophilic portions, and the criteria for selecting which amphiphilic substance to use are, for example, that it can solubilize only the target substance to be measured and not other substances that may be inhibitors. In other words, it is common sense for those skilled in the art to consider the effect of dispersing a water-insoluble substance in an aqueous solution by selecting an amphiphilic substance with a long hydrophobic portion, i.e., a high carbon number. Therefore, amphiphilic substances with short hydrophobic groups are not usually selected by those skilled in the art, because they are expected not to have the desired effect when added to reagents due to their weak hydrophobicity and difficulty in forming micelles of sufficient size. However, it was a surprising discovery that the addition of an amphiphilic substance having a short linear, branched, or cyclic saturated or unsaturated hydrocarbon group with 6 to 12 carbons, an aromatic hydrocarbon group with 6 to 12 carbons, or a steroid skeleton in a hydrophobic portion contributes to the high sensitivity of the reagents.

In addition to the amphiphilic substances mentioned above, chaotropic substances can also be added. Chaotropic substances that can be used in the present invention include, but are not limited to, urea. Amphiphilic substances are sometimes used for denaturing purposes such as protein solubilization, but in the present invention, as shown in the Examples below, the same effects as those of amphiphilic substances were obtained with chaotropic substances used as denaturing agents, and it is assumed that the denaturing effects contributed to the promotion of immune response.

The amphiphilic substances used in the present invention may be used in combination. It was surprising that when two or more types of amphiphilic substances were used in combination, the reactivity of the reagent showed the same or better effect even when the concentrations of the amphiphilic substances added to the reagent was less than that of the single amphiphilic substance. Amphiphilic substances added to the reagent are preferred because they can be carried out with less concentration than alone, since it is common sense for those skilled in the art to keep the concentration of the amphiphilic substance added to the reagent as low as possible, since too large a concentration may interfere with the main reaction, the immune reaction or the measurement of luminescence.

The amphiphilic substance added to the reagent should preferably be contained in the reaction solution at a concentration of 0.05% to 5.33%, more preferably 0.27% to 3.73%, and still more preferably 1.07% to 2.67%. This is a higher concentration than is normally added to reagents used in immune reactions. As already mentioned, when an amphiphilic substance having a short linear, branched, or cyclic saturated or unsaturated hydrocarbon group with 6 to 12 carbons, an aromatic hydrocarbon group with 6 to 12 carbons, or a steroid skeleton in the hydrophobic portion is used, the expected effect is small, and thus it is expected that a high concentration of amphiphilic substance must be added. Although it is presumed to be difficult to use as a reagent because bubbles are likely to be generated when used at a high concentration, the fact that it could be used without affecting the immune response due to the generation of bubbles was a surprising effect, as well as the effect of high sensitivity was sufficient.

The reaction solution may be defined as the reaction field where the reaction to form immune complexes between the substance to be measured and the immunological partner takes place, and can be either a homogeneous assay or a heterogeneous assay, which is distinguished by the presence or absence of a process to remove unreacted immunological partners and the substance to be measured by washing (B/F separation). The heterogeneous assay can be either a one-step method or a two-step method as described below.

The most suitable of these is selected to prepare the reaction solution for the antigen-antibody reaction. The combination of reaction solutions, reaction procedures, and reaction modes using the amphiphilic substance of the present invention can be tailored to the instruments used to perform the immunoassay method.

As a specific measurement method using the measurement reagent of the present invention, an immobilized antibody-antigen-labeled antibody complex is formed, for example, by reacting the immobilized antibody with the sample and reacting the labeled antibody at the same time (one-step method), or by reacting the labeled antibody after washing (two-step method). The unbound labeled antibody is then washed away and the amount of antigen in the sample can be determined from the amount of bound labeled antibody. Specifically, in the case of enzyme immunoassay, a substrate is reacted with the labeled enzyme under optimum conditions, and the amount of the reaction product is measured by an optical method or the like. In the case of fluorescence immunoassay, the fluorescence intensity of the fluorescent substance labeling is measured, and in the case of radioimmunoassay, the radiation dose of the radioactive substance labeling is measured. In the case of chemiluminescence immunoassay, the amount of luminescence emitted by the chemiluminescence reaction system is measured. These are methods for detecting the presence of a substance to be measured by forming an immune complex between a substance to be measured in a test sample and an antibody specific for the substance to be measured (antigen in the case of an antibody detection system) in a reaction solution and detecting a signal due to the formation of the immune complex as appropriate, including a step for performing measurement after removing unreacted sample and excess labeled substance by washing. The antibody to be used can be determined according to the assay system, but for highly sensitive quantitative measurement, a sandwich immunological measurement method (sandwich immunoassay) using two or more antibodies can be selected. The sandwich immunoassay method may be performed in one or more steps (two steps, three steps, or the like).

The reagent of the present invention may also include an instruction manual that includes instructions on the procedures for performing immunological assays using the reagent and precautions regarding storage, handling, or the like of the reagent itself.

In general, the treatment of choice for thrombosis is antiplatelet agents such as aspirin and the like for thrombosis in arteries such as myocardial infarction, cerebral infarction, and arteriosclerosis obliterans, where the disease progresses due to activation and aggregation of platelets, and anticoagulants such as warfarin, DOAC, and the like for venous thrombosis such as deep vein thrombosis, atrial fibrillation, cardiogenic cerebral embolism, and pulmonary embolism, where the disease progresses due to activation of the coagulation system in the atria. Although the present invention is intended for use in the examination and measurement in platelet-related thrombosis, platelets play an important role in thrombosis, which is mainly caused by the coagulation system, and the measurement of platelets may be useful. The present invention may enable accurate risk assessment of platelet-based thrombosis, which may lead to the selection of antiplatelet agents.

Therefore, measurement of blood sCLEC-2 concentration in patients with thrombosis or suspected thrombosis using the present invention will provide more reliable measurements not only in the diagnosis of thrombosis but also in monitoring the course of treatment, and will provide extremely useful information in the treatment of thrombosis.

### EXAMPLES

The present invention will now be further illustrated by, but is by no means limited to, the following Examples.

### <<Example 1: Preparation of sCLEC-2 measurement reagent and measurement>>

### (Preparation of reagent for measurement and preparation of test samples)

· Sample diluent liquid: 0.1 mol/L HEPES buffer (pH 7.5) containing a preservative was used.

The antibodies in the reagent were prepared as follows, using antibodies described in Examples of Japanese Patent No. 6078845.
· First antibody solution: A mouse monoclonal antibody (11D5) recognizing sCLEC-2 was carried on magnetic latex particles (JSR Corporation) and dispersed in 0.01 mol/L MES buffer (pH 6.0) containing a preservative.
· Second antibody solution: Another mouse monoclonal antibody (11E6) recognizing sCLEC-2 was labeled with alkaline phosphatase (ALP) by a maleimide method and dispersed in 0.01 mol/L MES buffer (pH 6.0) containing a preservative.
· Luminescent substrate solution: Disodium 2-chloro-5-(4-methoxyspiro{1,2-dioxetane-3,2'-(5'-chloro)-tricyclo[3.3.1.13,7]decane}-4-yl)-1-phenyl phosphate (CDP-Star (registered trademark): Applied Biosystems) was used.
· B/F washing buffer: A buffer solution containing 0.1 mol/L citric acid (pH 6.5), 0.15 mol/L NaCl, and 0.1% TritonX-100 was used.
· Test samples: A recombinant hCLEC-2 protein diluted with a buffer solution (0.025 mol/L HEPES, 0.14 mol/L NaCl, 0.1% sodium octanoate, and 0.3% BSA) was used as test sample 1, and the same diluted with citrate plasma was used as test sample 2.

### (Measurement by measurement reagent)

An automated clinical testing system STACIA (registered trademark, LSI Medience Corporation) was used for the measurement.

STACIA-dedicated bottles were filled with the prepared sample diluent liquid, the first antibody solution (magnetic latex reagent), and the second antibody solution (enzyme-labeled antibody reagent), respectively, and placed in the apparatus. The measurement was performed according to the operation method of the apparatus.

Specifically, 40 µL of the sample diluent liquid was added to 10 µL of the sample and warmed at 37°C for several minutes, and then 25 µL of the first antibody solution (magnetic latex reagent) was added and warmed at 37°C for several minutes. Then, B/F separation was performed, 50 µL of the second antibody solution (enzyme-labeled antibody reagent) was added, and the mixture was warmed at 37°C for several minutes. B/F separation was performed again, 100 µL of the luminescent substrate solution was added, and the signal intensity (counts) was measured after several minutes of reaction at 37°C.

### <<Example 2: Effect of surfactant addition to reaction liquid on the reactivity of sCLEC-2>>

The procedure was conducted as in Example 1, except that surfactants with different hydrophobic and hydrophilic structures were added to the sample diluent liquid to achieve the following concentrations in the reaction solution (75 µL) after the addition of the first antibody solution.

As those with long carbon chains (C12 or higher) in the hydrophobic portion, Tween 20: polyoxyethylene(20) sorbitan monolaurate, Triton X-100: polyoxyethylene octylphenyl ether, and Emulgen 120, Emulgen 150: polyoxyethylene lauryl ether were added to the sample diluent liquid to achieve concentrations of 05%, 0.27%, and 1.07%.

As those with short carbon chains (C8) in the hydrophobic portion, sodium octanoate was added to achieve concentrations of 0.05%, 0.27%, 1.07%, 2.67%, 3.73%, and 5.33%, and n-octyl-β-D-glucoside was added to achieve concentrations of 0.05%, 0.27%, and 1.07%, to the sample diluent liquid.

Table 1-1 shows the results of the measurement of test sample 1 and Table 1-2 shows the results of the measurement of test sample 2.

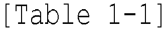

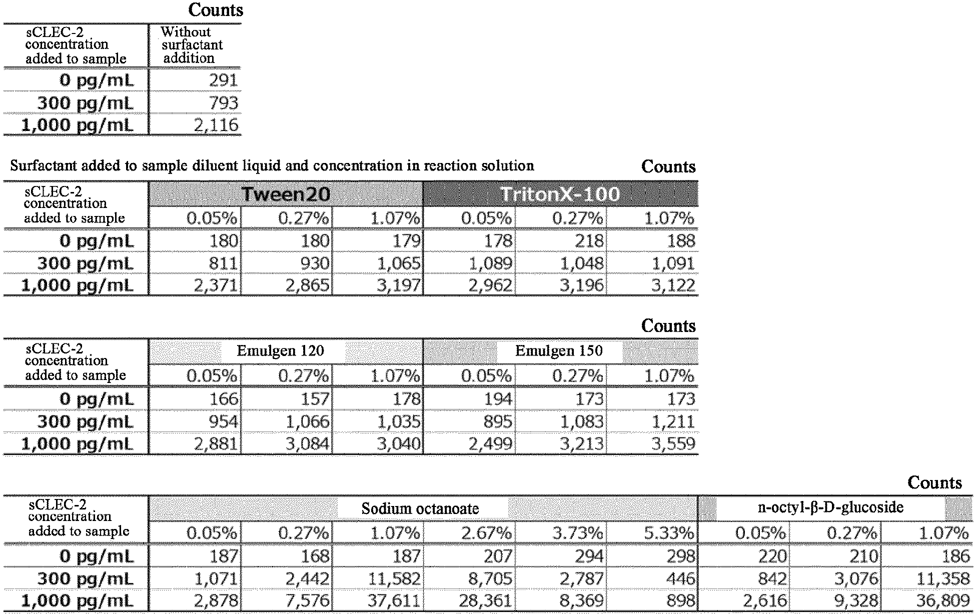

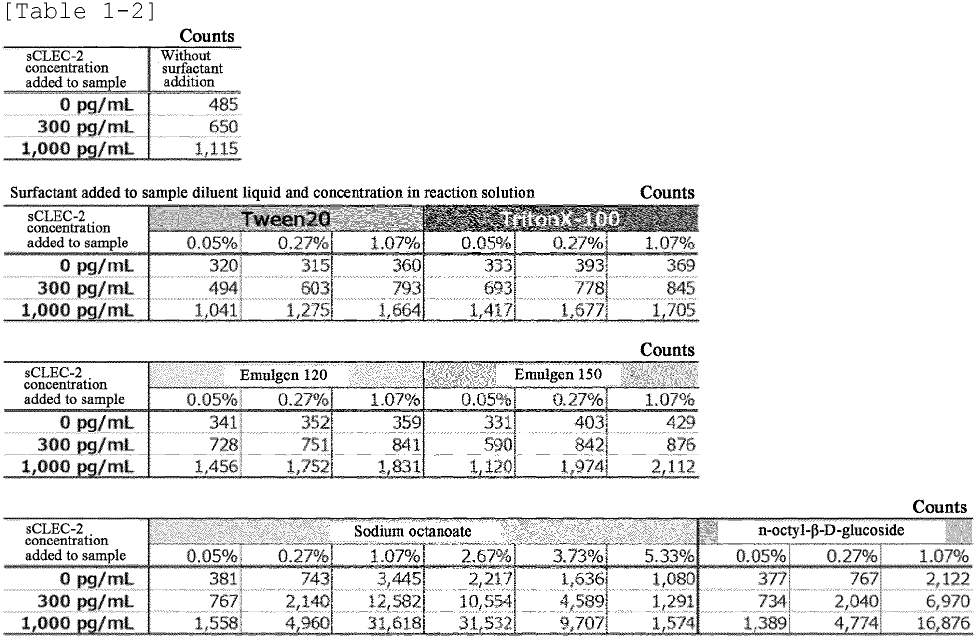

Compared to the results without surfactant addition, an increase in the measured values (counts) was observed under the conditions of sodium octanoate at 0.27% to 3.73% addition and n-octyl-β-D-glucoside at 0.27% and 1.07% addition. On the other hand, the addition of other surfactants did not increase the measured values (counts). In the sCLEC-2 measurement, it was shown that surfactants with a small molecular weight of the hydrophobic portion are effective in improving the reactivity.

### <<Example 3: Effect of the addition of surfactants with carbon chain shorter than C12 in hydrophobic portion to reaction liquid on the reactivity of sCLEC-2>>

The procedure was conducted according to the method of Example 2, except for the following.

Surfactants with different hydrophobic and hydrophilic structures shown in Table 2 were added to the sample diluent liquid to achieve concentrations of 0.27% and 1.07% in the reaction solution (75 µL) after the addition of the first antibody solution.

Table 3-1 shows the results of the measurement of test sample 1 and Table 3-2 shows the results of the measurement of test sample 2.

**[Table 2]**

| Surfactant | Number of carbons in hydrophobic portion | Type of hydrophilic portion |
|---|---|---|
| Sodium octanoate | 8 | Fatty acid |
| Sodium pelargonate | 9 | |
| n-Heptyl-β-D-thioglucoside (HTG) | 7 | Glucose-type pyranoside |
| n-Octyl-β-D-glucoside (OG) | 8 | |
| α-D-Glucopyranosyl-α-D-glucopyranoside monooctanoate (T-C8) | 8 | Trehalose ester-type pyranoside |
| α-D-Glucopyranosyl-α-D-glucopyranoside monodecanoate (T-C10) | 10 | |
| α-D-Glucopyranosyl-α-D-glucopyranoside monododecanoate (T-C 12) | 12 | |
| n-Octyl-β-D-maltoside (OM) | 8 | Maltose-type pyranoside |
| n-Nonyl-β-D-thiomaltoside (NTM) | 9 | |
| n-Decyl-β-D-maltoside (DM) | 10 | |
| n-Octanoyl-*N-*methylglucamine (MEGA-8) | 8 | Glucamine type |
| n-Nonanoyl-*N-*methylglucamine (MEGA-9) | 9 | |
| Tetraethylene glycol monooctyl ether (TEG-C8) | 8 | Oxyethylene type |
| Poly(ethylene glycol) octyl ether (PEG-C8) | 8 | |

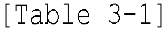

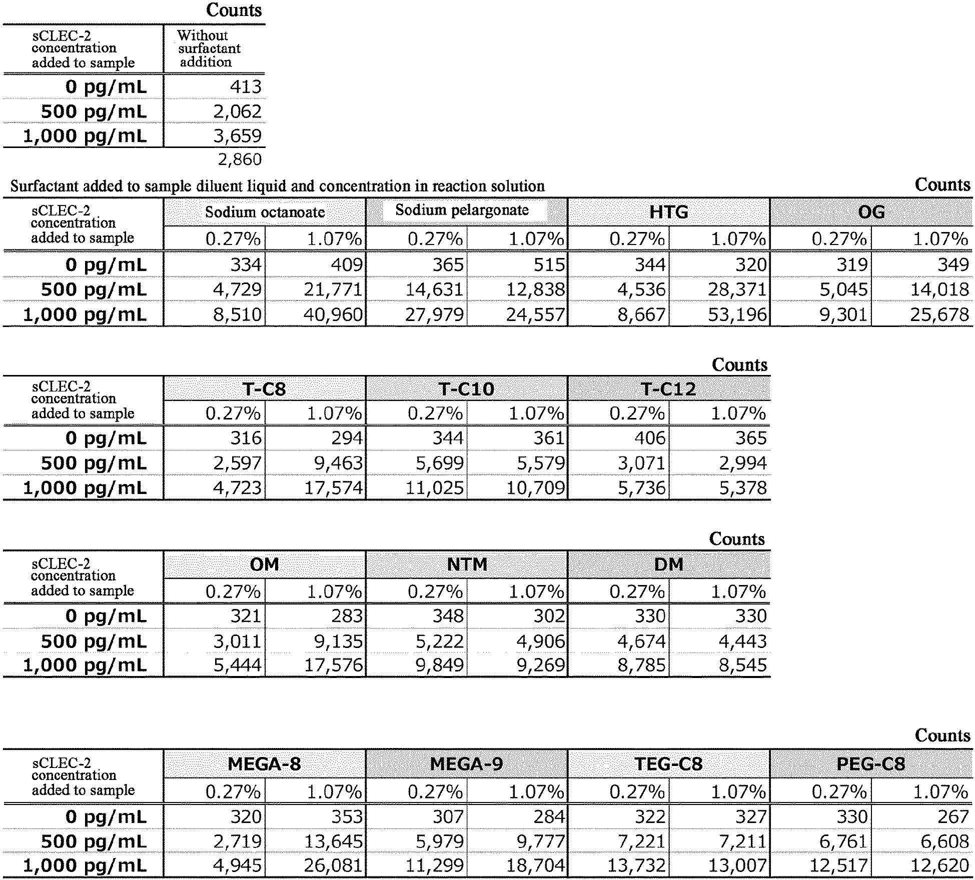

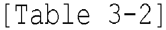

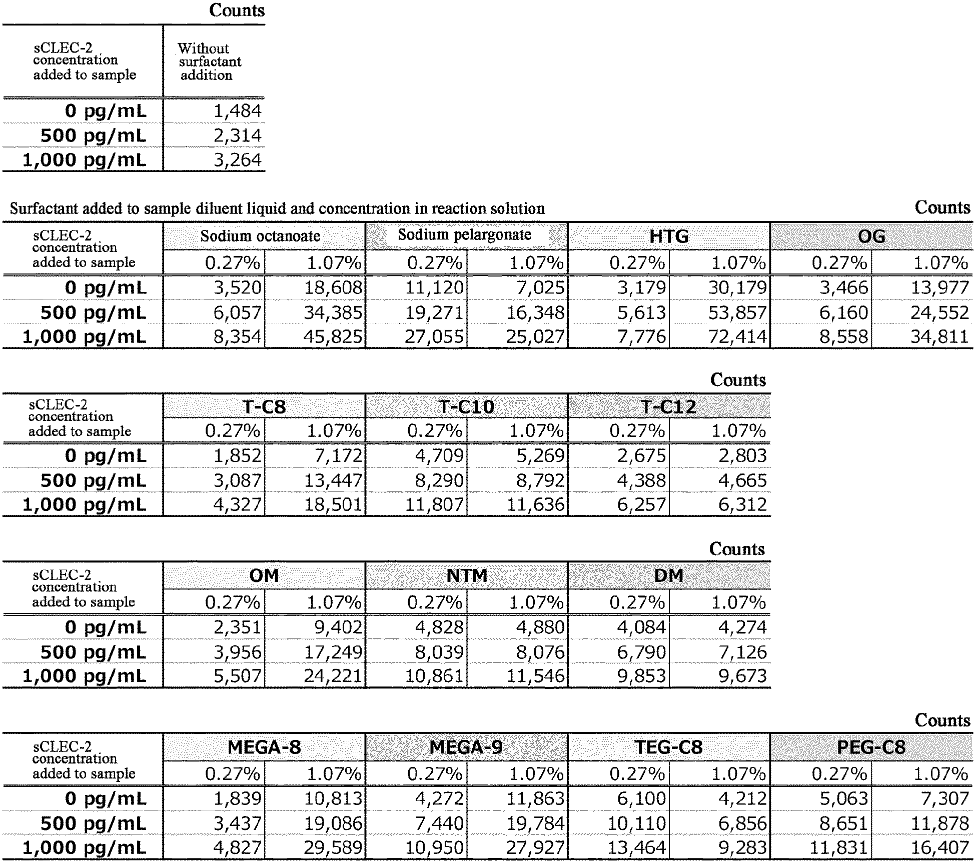

Compared to the results without surfactant addition, an increase in the measured values (counts) was observed under all conditions. The effects of HTG, sodium octanoate, MEGA-8, OG, and sodium pelargonate were particularly strong (more than 5-fold). The hydrophilic types with a small molecular weight, such as fatty acids, glucose type, and glucamine type, tended to be more effective.

### <<Example 4: Effect of the addition of sulfonates (C5 to C8) with different fatty acid carbon chain lengths in hydrophobic portion to reaction liquid on the reactivity of sCLEC-2>>

The procedure was conducted according to the method of Example 1, except for the following.

Sodium hexanesulfonate, sodium heptanesulfonate, and sodium octanesulfonate were added to the sample diluent liquid to achieve concentrations of 0.27%, 1.07%, and 2.67% in the reaction solution (75 µL) after the addition of the first antibody solution.

Table 4-1 shows the results of the measurement of test sample 1 and Table 4-2 shows the results of the measurement of test sample 2.

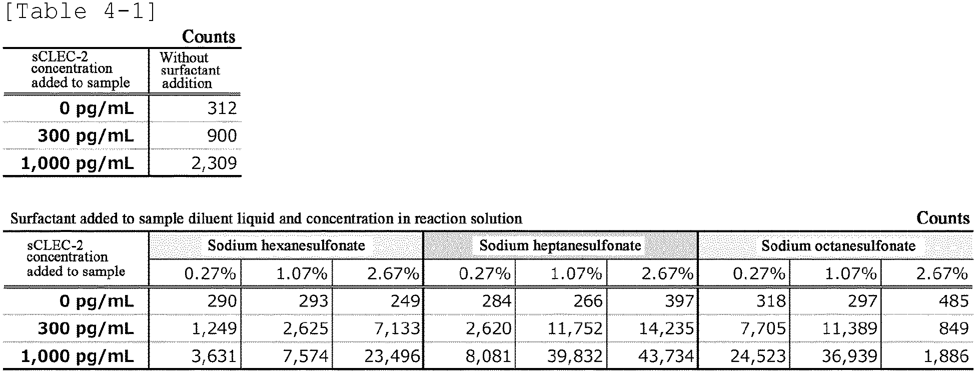

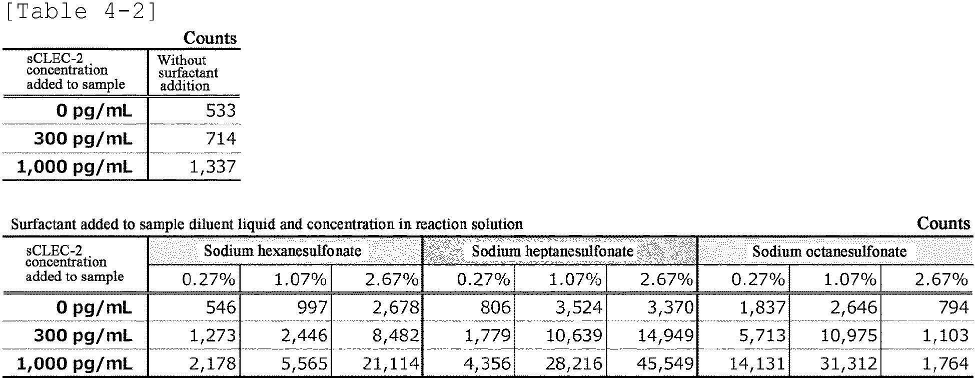

Compared to the results without surfactant addition, an increase in the measured values (counts) was observed under the conditions of sodium hexanesulfonate at 1.07% or more, sodium heptanesulfonate at 0.27% to 2.67%, and sodium octanesulfonate at 0.27% and 1.07% addition. The addition of sodium octanesulfonate at 2.67% did not increase the measured values (counts).

Next, sodium octanesulfonate was added to the sample diluent liquid to achieve concentrations of 0.05%, 0.27%, 1.07%, and 2.67%. Table 4-3 shows the results of the measurement of test sample 1 and Table 4-4 shows the results of the measurement of test sample 2. The same increase in the measured values (counts) was observed when 0.05% sodium octanesulfonate was added.

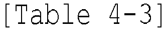

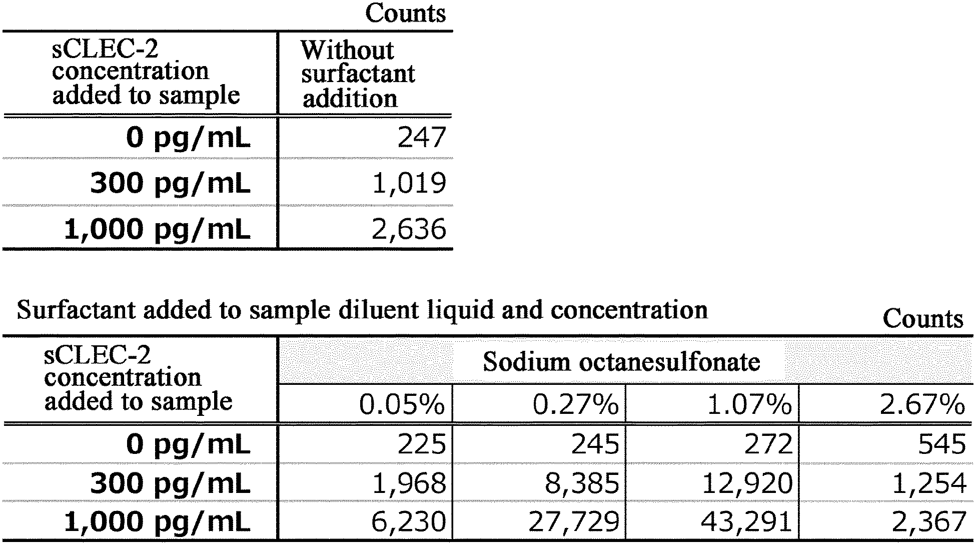

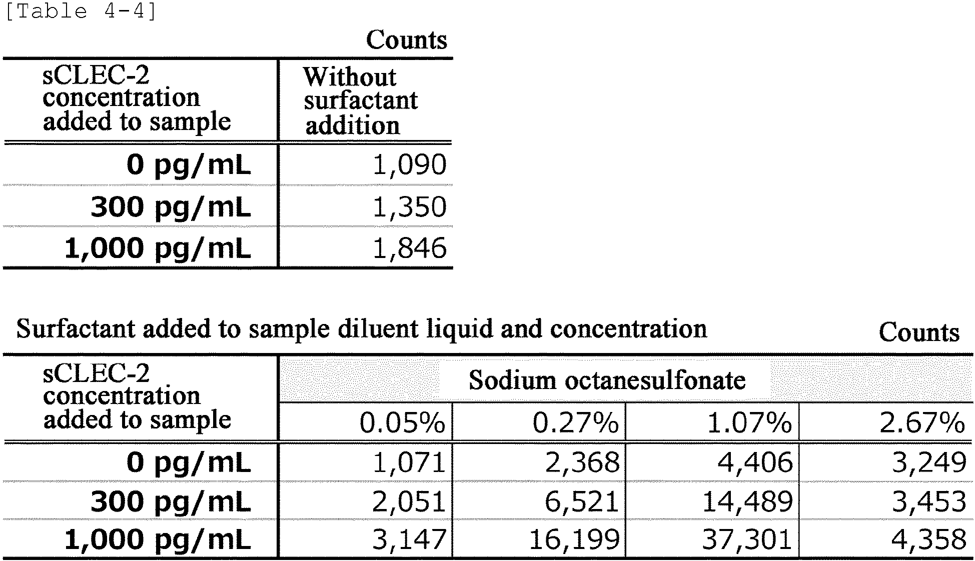

### <<Example 5: Effect of the addition of chaotropic substance, and surfactants with non-linear hydrophobic structures in hydrophobic portion to reaction liquid on the reactivity of sCLEC-2>>

The procedure was conducted according to the method of Example 2, except that a chaotropic substance and a surfactant were added to the sample diluent liquid to achieve the following concentrations in the reaction solution (75 µL) after the addition of the first antibody solution.

Urea as the chaotropic substance was added to the sample diluent to achieve concentrations of 0.27 mol/L, 1.1 mol/L, and 3.2 mol/L.

Sodium salicylate as a surfactant with a cyclic structure in the hydrophobic portion was added to the sample diluent liquid to achieve concentrations of 0.05%, 0.27%, 1.07%, and 2.67%.

Sodium cholate as a surfactant with a steroid skeleton in the hydrophobic portion, and CHAPS as an amphiphilic surfactant were added to the sample diluent to achieve concentrations of 0.05%, 0.27%, 1.07%, and 2.67%, respectively.

Table 5-1 shows the results of the measurement of test sample 1 and Table 5-2 shows the results of the measurement of test sample 2.

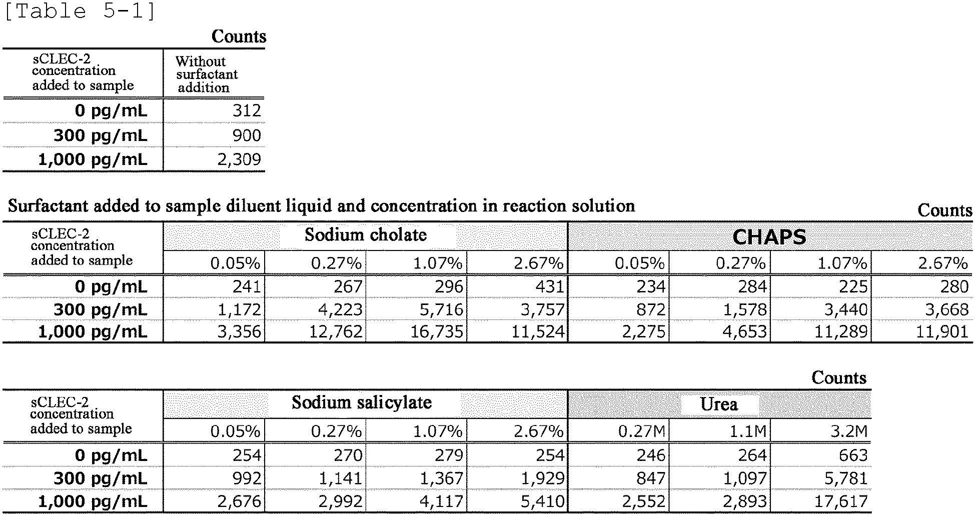

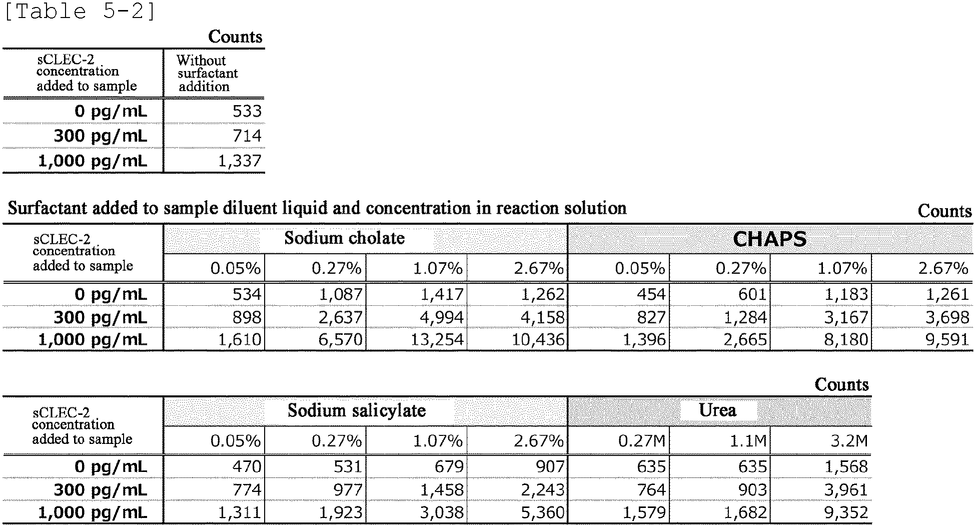

Compared to the results without surfactant addition, an increase in the measured values (counts) was observed under the conditions of sodium cholate and CHAPS at 0.27% or more, sodium salicylate at 2.67%, and urea at 3.2mol/L.

### <<Example 6: Reduction of the difference in sample matrices in sCLEC-2 measurement by combining the addition of various surfactants>>

The procedure was conducted according to the method of Example 2, except for the following.

As the concentrations in the reaction solution (75 µL) after the addition of the first antibody solution, 1.07% sodium octanoate or 1.07% sodium octanoate in combination with the following surfactants at concentrations of 0.27% and 1.07% were added to the sample diluent liquid:
n-Heptyl-β-D-thioglucoside (HTG), n-Octyl-β-D-glucoside (OG), and MEGA-9.

Table 6-1 shows the results of the measurement of test sample 1 and Table 6-2 shows the results of the measurement of test sample 2.

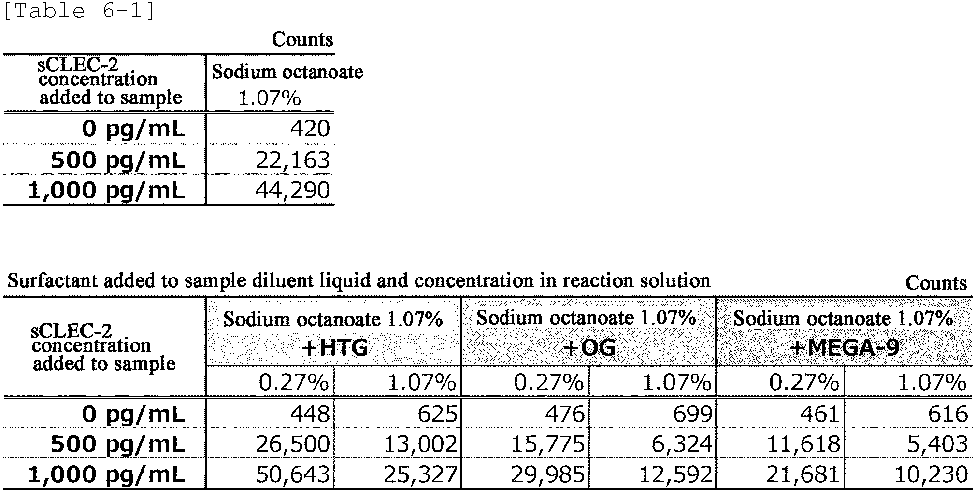

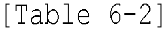

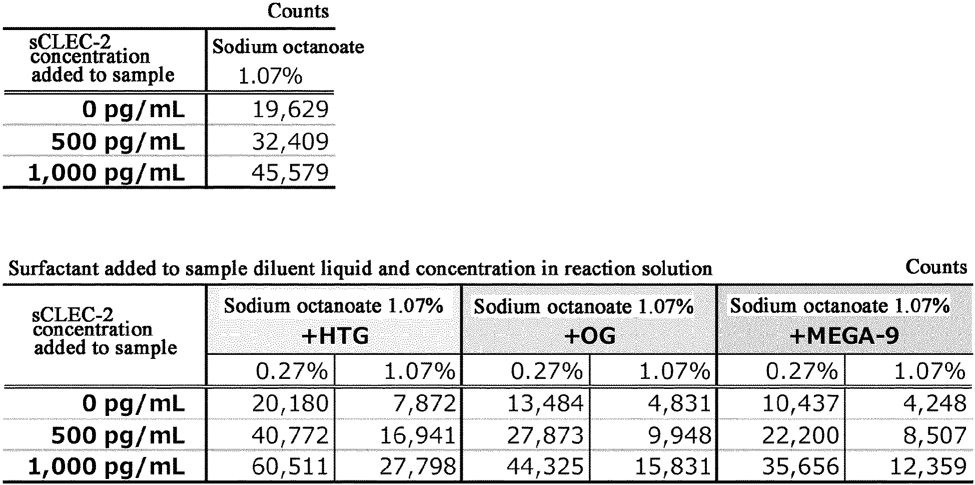

From the results of Table 6-1 and Table 6-2, the measured values (counts) of sCLEC-2 addition in test sample 1 are summarized in Table 6-3 and those of sCLEC-2 addition in test sample 2 are summarized in Table 6-4. Δcount was calculated as the difference between the measured value of each test sample without sCLEC-2 addition (0 pg/mL) and that of each sample with sCLEC-2 addition (500 pg/mL or 1,000 pg/mL).

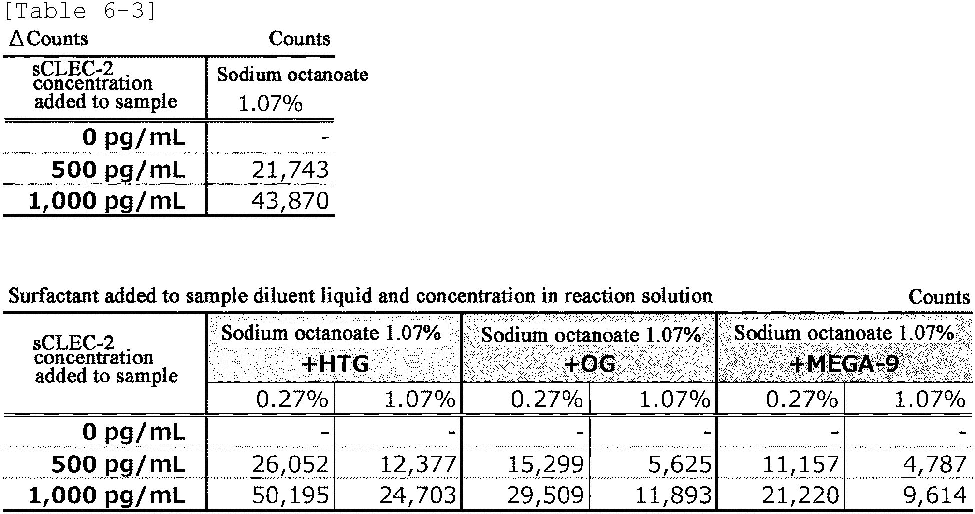

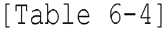

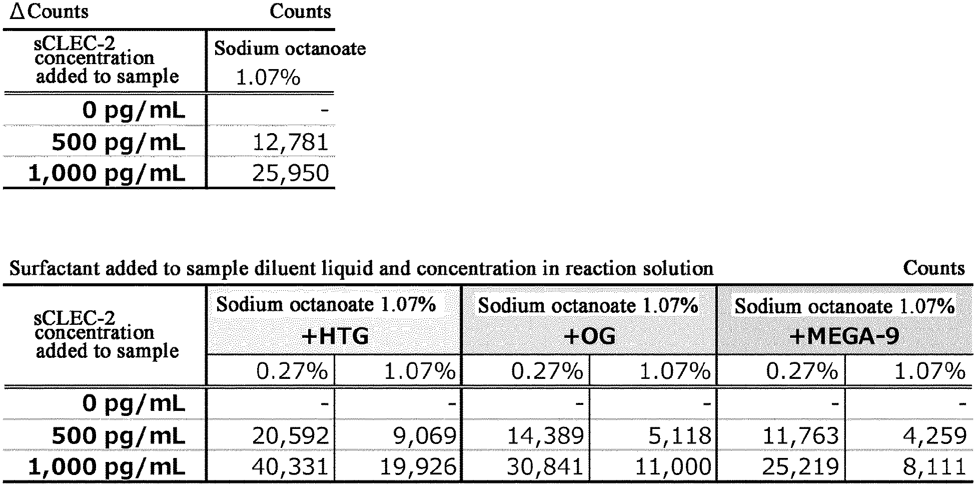

The reactivity of the sCLEC-2 measurement due to the difference in sample matrices was compared by dividing the results in Table 6-4 by the results in Table 6-3 under each measurement condition (Table 6-5).

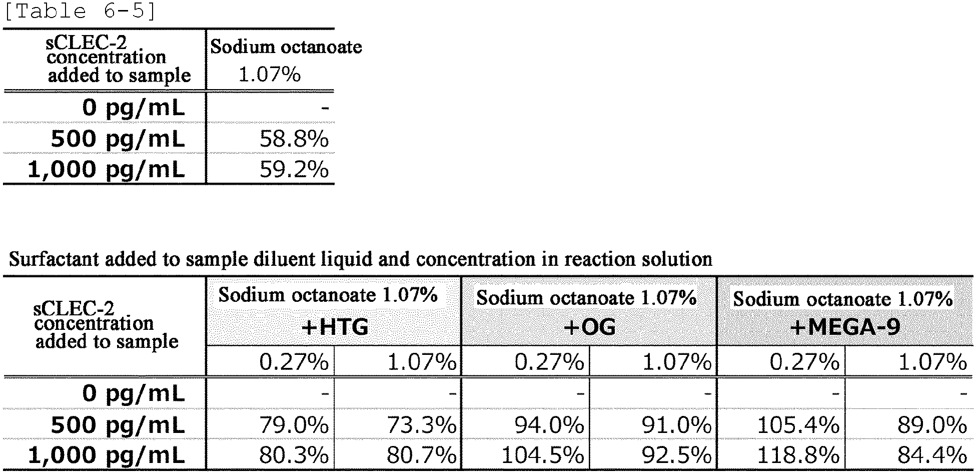

Compared to the condition with the addition of sodium octanoate alone, the combination of sodium octanoate with other surfactants reduced the difference in reactivity due to the difference in sample matrices. In the condition in combination with OG, the effect due to the difference in matrices was smaller. It was shown from this that multiple surfactants can be used in combination.

### <<Example 7: Sensitivity test>>

The procedure was conducted according to the method of Example 2, except for the following.

Sample diluent liquid: The combination of sodium octanoate and n-Octyl-β-D-glucoside (OG) was added to the sample diluent liquid to achieve concentrations of 1.07% and 0.27%, respectively, in the reaction solution (75 µL) after the addition of the first antibody solution.

Test samples: A recombinant hCLEC-2 protein was diluted to 0, 10, 25, 50, and 100 pg/mL using a buffer solution (0.025 mol/L HEPES, 0.14 mol/L NaCl, 0.1% sodium octanoate, and 0.3% BSA).

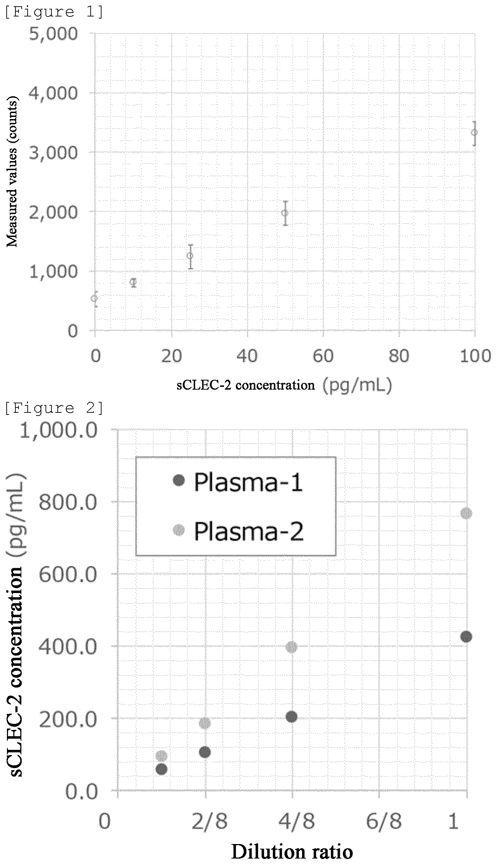

Test samples of each concentration were measured at a multiplicity of n=10, and the mean and standard deviation (SD) of the measured values (counts) were calculated and evaluated. The results are shown in Table 7 and Figure 1.

The +2SD of 0 pg/mL and the -2SD of 10 pg/mL did not overlap, indicating that 10pg/mL or more can be measured.

**[Table 7]**

| sCLEC-2 concentration pg/mL | **0** | **10** | **25** | **50** | **100** |
|---|---|---|---|---|---|
| **n=10** | 468 | 796 | 1,203 | 1,932 | 3,209 |
| | 554 | 797 | 1,207 | 1,869 | 3,453 |
| | 495 | 781 | 1,186 | 2,030 | 3,488 |
| | 548 | 846 | 1,130 | 2,061 | 3,319 |
| | 561 | 781 | 1,145 | 2,174 | 3,241 |
| | 604 | 821 | 1,279 | 1,939 | 3,341 |
| | 396 | 773 | 1,205 | 1,877 | 3,309 |
| | 554 | 877 | 1,490 | 1,894 | 3,354 |
| | 470 | 778 | 1,297 | 1,877 | 3,233 |
| | 551 | 785 | 1,243 | 2,030 | 3,190 |
| n=10 mean | 520 | 804 | 1,239 | 1,968 | 3,314 |
| 50 | 61 | 34 | 103 | 102 | 100 |
| CV | 11.8% | 4.3% | 8.3% | 5.2% | 3.0% |
| -2SD | 397 | 735 | 1,033 | 1,765 | 3,113 |
| +250 | 643 | 872 | 1,444 | 2,171 | 3,514 |

### <<Example 8: Dilution linearity test (CLEIA method) >>

The procedure was conducted according to the method of Example 2, except for the following.

Sample diluent liquid: The combination of sodium octanoate and n-Octyl-β-D-glucoside (OG) was added to the sample diluent liquid to achieve concentrations of 1.07% and 0.27%, respectively.

Standard sample: A recombinant hCLEC-2 protein was diluted to 0 and 500 pg/mL using a buffer solution (0.025 mol/L HEPES, 0.14 mol/L NaCl, 0.1% sodium octanoate, and 0.3% BSA). The sCLEC-2 concentrations of test samples were calculated by comparing them with the measured values (counts) of the standard sample.

Test samples: Two types of pooled plasma (Plasma-1: citrate-added plasma, Plasma-2: EDTA-added plasma) were diluted 2-fold, 4-fold, and 8-fold with physiological saline (Otsuka Pharmaceutical), respectively. Each test sample was measured in duplicate. The results are shown in Table 8 and Figure 2.

Good dilution linearity was observed from undiluted measurement without dilution to 8-fold dilution.

**[Table 8]**

| | **Dilution ratio** | sCLEC-2 concentration **(pg/mL)** | **vs. theoretical value** |
|---|---|---|---|
| **Plasma-1** | **1** | 422.6 | 100.0% |
| | **2** | 200.9 | 95.1% |
| | **4** | 101.6 | 96.2% |
| | **8** | 56.1 | 106.2% |
| **Plasma-2** | **1** | 762.1 | 100.0% |
| | **2** | 393.5 | 103.3% |
| | **4** | 182.2 | 95.6% |
| | **8** | 92.3 | 96.9% |

### <<Example 9: Distribution in healthy persons (CLEIA method) >>

Test samples: 5 mL of citrate-added plasma collected from healthy persons was centrifuged at 2,000 g for 15 minutes and used as the test samples.

The procedure was conducted according to the method of Example 2, except for the following.

Sample diluent liquid: The combination of sodium octanoate and n-Octyl-β-D-glucoside (OG) was added to the sample diluent liquid to achieve concentrations of 1.07% and 0.27%, respectively.

Standard sample: A recombinant hCLEC-2 protein was diluted to 0 and 300 pg/mL using a buffer solution (0.025 mol/L HEPES, 0.14 mol/L NaCl, 0.1% sodium octanoate, and 0.3% BSA). The sCLEC-2 concentrations of test samples were calculated by comparing them with the measured values (counts) of the standard sample. The results are shown as "STACIA sCLEC-2".

The results are shown in Table 9.

All obtained sCLEC-2 measurement results were equal to or more than the analytical sensitivity shown in Example 7.

The mean, standard deviation, and median values were 59.1, 16.7, and 54.6 pg/mL, respectively, and the reference standard range, expressed as the 2.5th percentile to 97.5th percentile values, was 34.0 to 101.7 pg/mL.

**[Table 9]**

| STACIA sCLEC-2 | | | (pg/mL) |
|---|---|---|---|
| | mean | SD | median |
| **Total** | 59.1 | 16.7 | 54.6 |
| **Male** | 62.4 | 17.2 | 57.9 |
| **Female** | 51.3 | 12.2 | 51.5 |

| Quantile | | sCLEC-2 (pg/mL) | |
|---|---|---|---|
| | 0.025 | 34.0 | |
| | 0.975 | 101.7 | |
| | Median | 54.6 | |

When using measurement methods and reagents as clinical tests, it is necessary to set a cutoff value to discriminate pathological conditions. For this, a range that is considered normal based on the reference range is defined. However, in the case of measurement methods and reagents with large dispersion values, the range of confidence intervals is wide, and the reference range derived from such samples must be said to be unreliable, and as a result, the set cutoff values are also unreliable. Therefore, the measurement by the ELISA assay is not sensitive enough and does not provide reliable results in terms of the accuracy of the measurement itself.

### <<Example 10: Effect of the addition of surfactants with cationic functional group, amphiphilic functional group, or branched structure in hydrophobic portion to reaction liquid on the reactivity of sCLEC-2>>

The procedure was conducted according to the method of Example 2, except for the following.

The procedure was conducted according to the method of Example 2, except that the following surfactants with different hydrophobic and hydrophilic structures were added to the sample diluent liquid to achieve the following concentrations in the reaction solution after the addition of the first antibody solution.

n-Octyltrimethylammonium chloride was added to the sample diluent liquid as a surfactant with a cationic functional group at 0.05%, 0.27%, 1.07% and 2.67%.

Dimethyl(n-octyl)(3-sulfopropyl)ammonium hydroxide intramolecular salt or decyldimethyl(3-sulfopropyl)ammonium hydroxide intramolecular salt as a surfactant with an amphiphilic functional group was added to the sample diluent liquid to achieve concentrations of 0.05%, 0.27%, 1.07% and 2.67%.

Sodium 2-ethylhexyl sulfate as a surfactant with a branched hydrophobic structure was added to the sample diluent liquid to achieve concentrations of 0.05%, 0.27%, 1.07% and 2.67%.

Table 10-1 shows the results of the measurement of test sample 1 and Table 10-2 shows the results of the measurement of test sample 2.

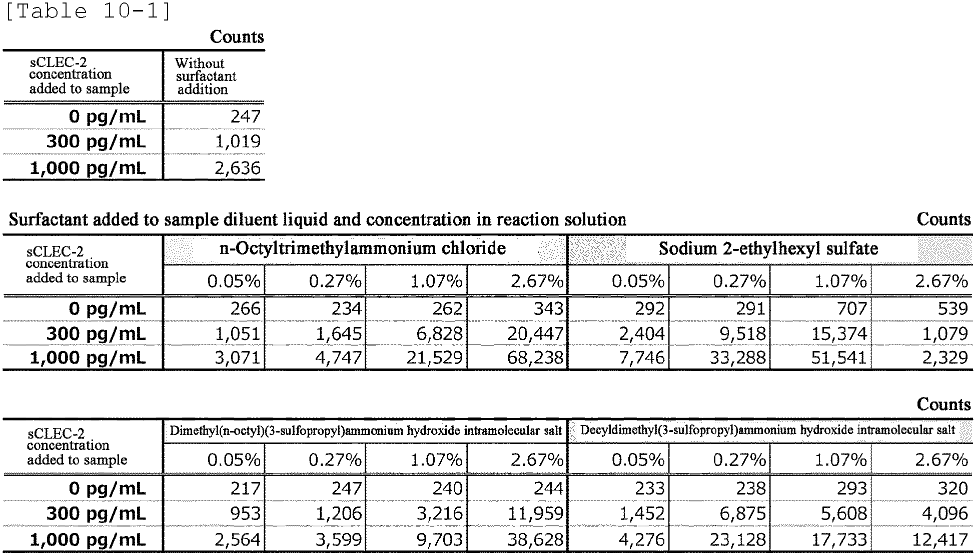

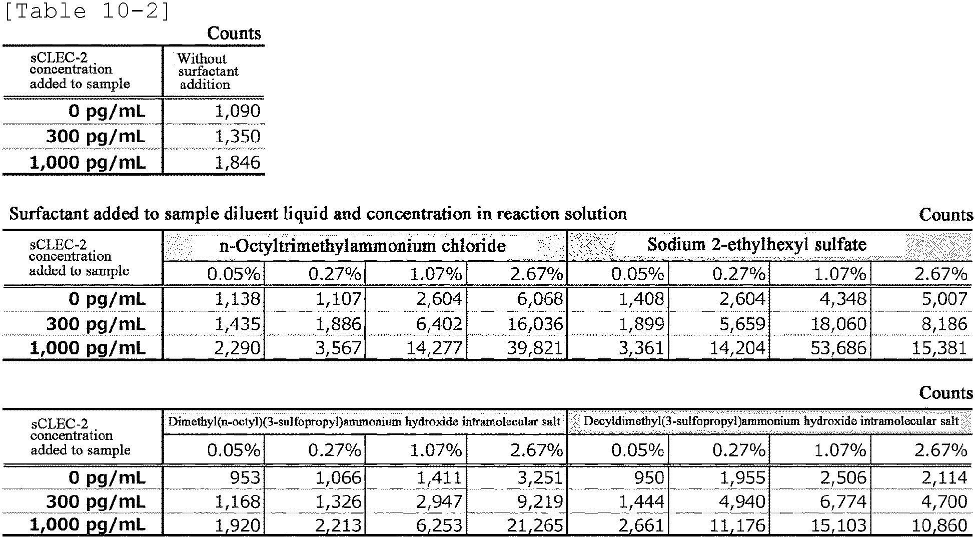

Compared to the results without surfactant addition, an increase in the measured values (counts) was observed under the conditions of n-octyltrimethylammonium chloride at 0.27% to 2.67%. An increase in the measured values (counts) was observed under the conditions of dimethyl(n-octyl)(3-sulfopropyl)ammonium hydroxide intramolecular salt at 1.07% to 2.67%. For decyldimethyl(3-sulfopropyl)ammonium hydroxide intramolecular salt, an increase in the measured values (counts) was observed at 0.05% to 1.07%, but an increase in the measured values (counts) of test sample 1 was not observed at 2.67%.

### INDUSTRIAL APPLICABILITY

By using the measurement reagent and the measurement method of the present invention, highly sensitive measurement is possible without being affected by interfering substances. Therefore, it is possible to accurately measure the concentration of the substance to be measured in the blood of healthy persons. Therefore, the measurement of concentrations can be used not only for accurate diagnosis, but also for selecting appropriate treatment methods, monitoring treatment effects, and predicting prognosis, thereby providing highly reliable measurement values and extremely useful information for medical care.

## Claims

1. A reagent for measuring a physiologically active substance, comprising:
a carrier on which a first immunological partner that binds to the physiologically active substance contained in a biological sample is immobilized;
a second immunological partner that binds to the physiologically active substance; and
an amphiphilic substance that contains, as a hydrophobic portion, a linear, branched, or cyclic saturated or unsaturated hydrocarbon group with 6 to 12 carbons, an aromatic hydrocarbon group with 6 to 12 carbons, or a steroid skeleton; or a chaotropic substance.

2. The reagent according to claim 1, wherein the amphiphilic substance that contains, as a hydrophobic portion, a linear, branched, or cyclic saturated or unsaturated hydrocarbon group with 6 to 12 carbons, an aromatic hydrocarbon group with 6 to 12 carbons, or a steroid skeleton is a compound that contains any one of an anionic functional group, a cationic functional group, an amphiphilic functional group, a pyranoside group, a glucamine group, an oxyethylene group, or an aromatic ring which may be substituted.

3. The reagent according to claim 1 or 2, wherein two or more of the amphiphilic substances, or a combination of the amphiphilic substance with the chaotropic compound, is used.

4. The reagent according to any one of claims 1 to 3, wherein a concentration of the amphiphilic substance in a reaction liquid is 0.05% to 5.33%, and a concentration of the chaotropic compound in the reaction liquid is 0.27 mol/L to 3.2 mol/L.

5. The reagent according to any one of claims 1 to 4, wherein an item to be measured is soluble CLEC-2.

6. A method of measuring a physiologically active substance contained in a biological sample using the reagent according to any one of claims 1 to 5, comprising:
(1) reacting the physiologically active substance contained in the biological sample with the carrier on which the first immunological partner that binds to the physiologically active substance contained in the biological sample is immobilized, and the second immunological partner that binds to the physiologically active substance, in the presence of the amphiphilic substance;
(2) separating the carriers from substances other than the carriers in a reaction liquid after step (1); and
(3) measuring the second immunological partner on the carrier that is separated in step (2).
